# EUROPÄISCHE PATENTSCHRIFT

(11) **EP 2 216 657 B1**
(45) Veröffentlichungstag und Bekanntmachung des Hinweises auf die Patenterteilung: **10.08.2011**
(21) Anmeldenummer: 09450230.9
(22) Anmeldetag: 04.12.2009
(51) Int. Cl.: G01S 19/22, G01S 19/28

(54) **Verfahren und Anlage zur Positionsbestimmung**
Positioning assembly and method
Procédé et installation de détermination de la position

(30) Priorität: 04.02.2009 AT 1912009
(43) Veröffentlichungstag der Anmeldung: 11.08.2010
(73) Patentinhaber: Riegl Laser Measurement Systems GmbH, 3580 Horn (AT)
(72) Erfinder: Ullrich, Andreas, 3003 Gablitz (AT); Rieger, Peter, 3824 Grossau (AT)
(74) Vertreter: Weiser, Andreas

(56) Entgegenhaltungen:
- DE-A1-102004 028 736
- JP-A- 2005 114 601
- JUN-ICHI MEGURO ET AL: "GPS accuracy improvement by satellite selection using omnidirectional infrared camera" INTELLIGENT ROBOTS AND SYSTEMS, 2008. IROS 2008. IEEE/RSJ INTERNATIONAL CONFERENCE ON, IEEE, PISCATAWAY, NJ, USA, 22. September 2008 (2008-09-22), Seiten 1804-1810, XP031348241 ISBN: 978-1-4244-2057-5
- SOLOVIEV A ET AL: "Utilizing multipath reflections in deeply integrated GPS/INS architecture for navigation in urban environments" POSITION, LOCATION AND NAVIGATION SYMPOSIUM, 2008 IEEE/ION, IEEE, PISCATAWAY, NJ, USA, 5. Mai 2008 (2008-05-05), Seiten 383-393, XP031340917 ISBN: 978-1-4244-1536-6

## Beschreibung

Die vorliegende Erfindung betrifft ein Verfahren und eine Anlage gemäβ den Oberbegriffen der Ansprüche 1 und 5.

Satellitennavigationssysteme (global navigation satellite systems, GNSS) wie das GPS-, GLONASS- oder GALILEO-System beruhen auf Entfernungsmessungen zu Satelliten mit bekannten Positionen anhand von Laufzeitmessungen der Sendesignale der Satelliten. Die Sendesignale enthalten jeweils Information über ihre Absendeposition und -zeit, sodaß bei bekannter Systemzeit aus einem Vergleich der Absendezeit mit der Empfangszeit im Empfänger die Signallaufzeit und damit die Satellitenentfernung bestimmt werden kann.

Die Genauigkeit der Laufzeitmessungen und damit der Positionsbestimmung wird durch zahlreiche Effekte beeinträchtigt, wie veränderliche Ausbreitungsbedingungen in der Atmosphäre, Umweg- oder Mehrwegeempfang (multipath reception). Umweg- bzw. Mehrwegeempfang kann beispielsweise durch Reflexion des Satellitensignals an Häusern oder Gebirgen auftreten, durch Brechung des Signals in der Ionosphäre, durch Streuung an kleinen Objekten wie Schildern oder Bäumen oder durch Beugung z.B. an Hauskanten oder Berggraten. Diese Effekte verlängern einerseits die Signallaufzeit, anderseits können sich durch Überlagerung von über verschiedene Wege empfangenen Teilen ein und desselben Sendesignals Datenfehler ergeben, welche die Entfernungsbestimmung verfälschen. So wird z.B. im GPS-System die Laufzeit mittels Phasenkorrelation eines im Sendesignal enthaltenen Bitmusters bestimmt, welches bei Eigenüberlagerung ein fälschlicherweise zeitversetzt korrelierbares Bitmuster ergeben kann.

Für sicherheitskritische und/oder hochpräzise Anwendungen wie die Luftfahrt oder die Vermessungstechnik ist daher eine selbständige Erkennung fehlerhafter Satellitensignale im Empfänger ("receiver autonomous integrity monitoring", RAIM), insbesondere mittels "fault detection and exclusion" (FDE), von entscheidender Bedeutung. Bekannte FDE-Verfahren verwenden beispielsweise eine statistische Auswertung der Empfangsvergangenheit zur Plausibilitätsüberprüfung der aktuell empfangenen Sendesignale, mit wechselhaftem Erfolg.

Aus der JP 2005/114601 A ist ein Verfahren zur GPS-Positionsbestimmung bekannt, bei welchem mittels eines Radarscanners für jeden von einer vorläufigen Position aus empfangbaren GPS-Satelliten überprüft wird, ob die "Line-of-Sight" zu diesem Satelliten blockiert ist oder nicht, und nur wenn nicht, dieser Satellit für die GPS-Bestimmung herangezogen wird.

Die Erfindung setzt sich zum Ziel, ein Verfahren und eine Anlage zum Erzeugen eines georeferenzierten Laserscanner-3D-Reflexionsbildes zu schaffen, welche eine verbesserte Georeferenzierung des 3D-Reflexionsbildes ermöglicht.

Dieses Ziel wird in einem ersten Aspekt der Erfindung mit einem Verfahren der einleitend genannten Art erreicht, das sich auszeichnet durch die Schritte

Bestimmen einer vorläufigen Position des Empfängers,

Aussenden eines Laserstrahls von der vorläufigen Position in Richtung zumindest eines empfangbaren Satelliten und Feststellen, ob der Laserstrahl reflektiert wird, und

Bestimmen der Position des Empfängers durch Auswerten der Sendesignale nur jener Satelliten, in deren Richtung keine Reflexion festgestellt wird,
wobei das Aussenden des Laserstrahls und Feststellen einer Reflexion durch das genannte Laserscannen des Umgebungsbereichs erfolgt, um ein auf die vorläufige Position bezogenes 3D-Reflexionsbild des Umgebungsbereichs zu erzeugen, in welchem anschließend festgestellt wird, ob die genannte Reflexion in Richtung eines Satelliten vorliegt, und
wobei das 3D-Reflexionsbild nach dem Bestimmen der Position auf diese georeferenziert wird.

- 2a -

Die Erfindung beruht auf dem neuartigen Ansatz, das beim 3D-Laserscannen anfallende 3D-Reflexionsbild gleichzeitig zur Überprüfung der Sichtbarkeit der Satelliten für eine verbesserte Georeferenzierung des 3D-Reflexionsbildes mitzuverwenden. Im Ergebnis wird auf überraschend einfache und zuverlässige Art und Weise eine hohe Positionsgenauigkeit der Georeferenzierung des erzeugten 3D-Reflexionsbildes erreicht.

Darüber hinaus eröffnet das Laserscannen eines ganzen Umgebungsbereichs, welcher ja eine Vielzahl potentieller Satellitenpositionen umfaßt, auch die Möglichkeit, das Verfahren in einen Vor-Ort-Erfassungsabschnitt und einen Post-Processing-Auswerteabschnitt aufzuteilen, um die vor Ort erforderliche Rechenleistung zu minimieren. Demgemäß besteht eine weitere bevorzugte Verfahrensvariante darin, das Auswerten des 3D-Reflexionsbildes und Bestimmen der Position in einer von den vorherigen Schritten abgesonderten Nachverarbeitung durchzuführen.

Der Laserstrahl kann auf jede in der Technik bekannte Art erzeugt und auf den zu überprüfenden Satelliten ausgerichtet werden, beispielsweise mit Hilfe einer mechanischen Schwenkvorrichtung. Besonders vorteilhaft ist es, wenn das Aussenden des Laserstrahls und Feststellen einer Reflexion mit Hilfe eines 3D-Laserscanners erfolgt, wie er aus der Vermessungstechnik an sich bekannt ist und für die Zwecke der Erfindung mitverwendet werden kann. Ein solcher 3D-Laserscanner kann dabei sowohl zum wahlfreien Ausrichten des Laserstrahles auf eine bestimmte Satellitenposition als auch zum Vor-Ort-Erstellen des 3D-Reflexionsabbildes der Umgebung eingesetzt werden.

Auch das Bestimmen der vorläufigen Position kann auf jede in der Technik bekannte Art und Weise erfolgen, beispielsweise mit Hilfe "grober" Positionsbestimmungsverfahren, wie einer Funkortung oder Zellerkennungen in Mobilfunknetzen, einer Bilderkennung von Referenzobjekten in der Umgebung usw. Besonders günstig ist es, wenn zur Vereinfachung auch die vorläufige Position mit Hilfe desselben Satellitennavigationssystems bestimmt wird, d.h. durch Auswerten der Sendesignale zunächst aller empfangbaren Satelliten.

In einem zweiten Aspekt schafft die Erfindung eine Anlage mit den Merkmalen des Anspruchs 5.

Bevorzugt ist die Anlage mit einem Speicher versehen, welcher die vom Empfänger empfangenen Sendesignale und die vom Laserscanner erzeugten 3D-Reflexionsbilder für eine spätere gemeinsame Auswertung im Empfänger protokolliert.

Besonders günstig ist es, wenn der Laserscanner ein 3D-Laserscanner ist, welcher mit einer Einrichtung zur Messung seiner Orientierung ausgestattet ist.

Hinsichtlich der Vorteile der erfindungsgemäßen Anlage und ihrer verschiedenen Ausführungsformen wird auf die obigen Ausführungen zu den entsprechenden Aspekten des Verfahrens und die nachstehende Beschreibung bevorzugter Ausführungsbeispiele der Erfindung verwiesen, welche auf die begleitenden Zeichnungen Bezug nimmt, in denen zeigt:
Fig. 1 ein globales Satellitennavigationssystem in einem schematischen Überblick;
Fig. 2 die Anlage der Erfindung in einer beispielhaften Umgebung in einer nicht-maßstäblichen Perspektivansicht;
Fig. 3 ein Flußdiagramm einer ersten Ausführungsform des Verfahrens der Erfindung; und
Fig. 4 ein Flußdiagramm einer zweiten Ausführungsform des Verfahrens der Erfindung.

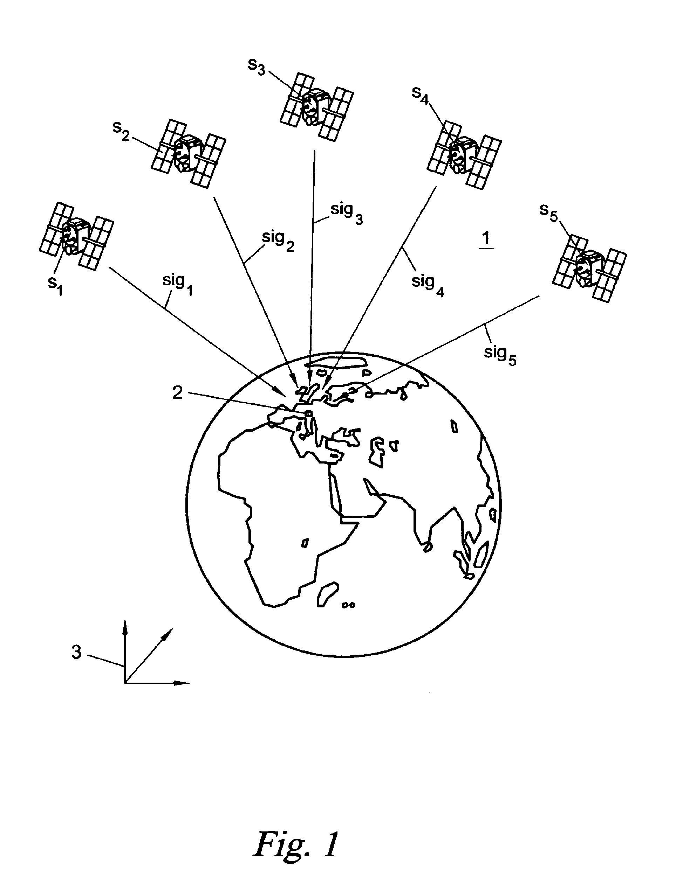

Gemäß Fig. 1 umfaßt ein globales Satellitennavigationssystem (GNSS) 1 eine Vielzahl erdumlaufender Satelliten sᵢ, von denen stellvertretend fünf Satelliten s₁ - s₅ gezeigt sind. Jeder Satellit sᵢ sendet fortlaufend, insbesondere periodisch, Sendesignale sigᵢ (i= 1..5), die von einem Satellitennavigationsempfänger 2 auf der Erde empfangen werden.

Jedes Sendesignal sigᵢ enthält eine eindeutige Kennung idᵢ des Satelliten sᵢ sowie die Zeit tsᵢ und Position posᵢ des Satelliten sᵢ zum Zeitpunkt der Aussendung des Sendesignals sigᵢ. Wie dem Fachmann bekannt, wird in der Praxis die Position posᵢ verallgemeinert in Form der Ephemeridendaten des Satelliten sᵢ übermittelt, aus denen anhand der Sendezeit tsᵢ die Sendeposition posᵢ abgeleitet werden kann.

Aus dem gesamten Satz "set" aller an einer bestimmten Position "pos" zu einem bestimmten Zeitpunkt vom Empfänger 2 empfangenen Sendesignale sigᵢ werden durch Messen der Signallaufzeiten, die sich aus einem Vergleich der jeweiligen Sendezeit tsᵢ mit der aktuellen Empfangszeit ergeben, die Entfernungen zu den Satelliten sᵢ und daraus die Position pos des Empfängers 2 im Referenzkoordinatensystem 3 des Satellitennavigationssystems 1 bestimmt, wie in der Technik bekannt.

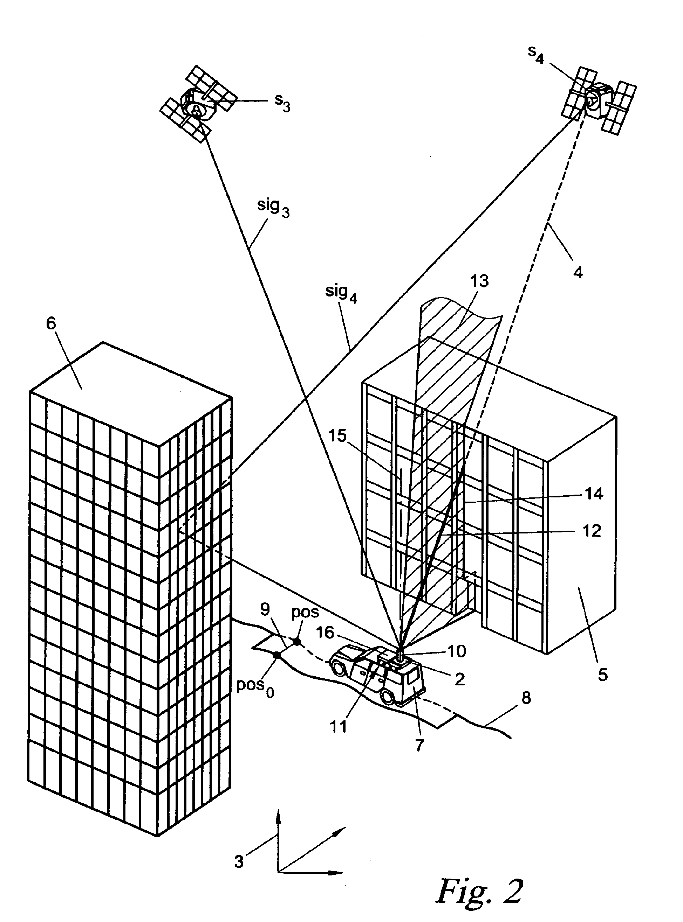

Im Falle eines Umweg- oder Mehrwegeempfangs eines Sendesignals sigᵢ kann es zu Interpretationsfehlern dieses Sendesignals im Empfänger 2 kommen. Fig. 2 zeigt das einfache Beispiel einer Abschattung der Sichtlinie 4 zwischen dem Satelliten s₄ und dem Empfänger 2 durch ein Hochhaus 5 und einer Reflexion an einem gegenüberliegenden Hochhaus 6, wodurch sich ein Laufzeitund damit ein Entfernungsmeßfehler zum Satelliten s₄ ergibt. Es versteht sich, daß der Entfernungsmeßfehler auch auf andere Weise als durch die dargestellte Einfachreflexion hervorgerufen sein kann, beispielsweise durch Mehrwegeempfang mit Überlagerung mehrerer Versionen ein und desselben Sendesignals sigᵢ am Ort des Empfängers 2, z.B. durch Streuungs-, Beugungs-, Brechungs- oder Reflexionseffekte usw.

Der Empfänger 2 ist in dem gezeigten Beispiel - obwohl dies nicht zwingend ist - mobil und am Dach eines Fahrzeugs 7 montiert, welches zu Vermessungszwecken den Straßenzug zwischen den Hochhäusern 5 und 6 abfährt. Die Bewegungsbahn bzw. Trajektorie 8 des Fahrzeugs 7 wird dabei vom Empfänger 2 als Folge von Positionen pos bestimmt. Der beim Passieren des Hochhauses 5 durch Abschattung und Reflexion des Sendesignals sig₄ hervorgerufene Positionsmeßfehler äußert sich z.B. in einem vorübergehenden Versatz 9 der Trajektorie 8 zwischen den korrekten Positionen pos und fehlerbehafteten Position pos₀.

Um derartige Positionsmeßfehler auszuschließen, wird die folgende Anordnung eingesetzt. Der Empfänger 2 ist mit einem Laserscanner 10 zu einer Anlage 11 zur fehlerkorrigierten Positionsbestimmung kombiniert. Der Laserscanner 10 kann ein eigens zu diesem Zweck in die Anlage 11 integrierter Laserscanner oder aber ein Laserscanner sein, der bereits zu Vermessungszwecken auf dem Fahrzeug 7 montiert ist, beispielsweise für die Landvermessungen mittels Mobile Scanning bzw. Mobile Mapping.

Der Laserscanner 10 ist im einfachsten Fall ein 1D-Scanner, d.h. ein Laserentfernungsmesser, welcher in der Lage ist, einen Laserstrahl 12 auf ein Ziel zu richten und mittels eines integrierten optischen Detektors eine Reflexion des Laserstrahls 12 zu detektieren. Der Laserscanner 10 wird zur Überprüfung des Signals sig₄ in der präsumptiven Sichtlinie 4 zum Satelliten s₄ ausgerichtet, welche sich aus der vorläufig bestimmten Position pos₀ des Empfängers 2 und der im Sendesignal sig₄ angegebenen Position pos₄ des Satelliten s₄ ergibt.

Wird eine Reflexion des Laserstrahls 12 festgestellt, liegt ein Hindernis - wie hier das Hochhaus 5 - in der Sichtlinie 4, und das Sendesignal sig₄ des Satelliten s₄ wird als fehlerbehaftet eingestuft und von der Positionsbestimmung ausgeschlossen: Mit anderen Worten wird die korrigierte Position pos auf Grundlage eines bereinigten Satzes set von Sendesignalen {sig₁, sig₂, sig₃, sig₅} bestimmt, welcher das fehlerhafte Sendesignal sig₄ nicht mehr enthält.

Das genannte Ausrichten des Laserstrahls 12 auf einen Satelliten sᵢ kann beispielsweise mit Hilfe einer steuerbaren 2-Achsen-Servolagerung des Laserscanners 10 durchgeführt werden. Bevorzugt wird jedoch für den Laserscanner 10 ein 3D-Laserscanner eingesetzt. Ein 3D-Laserscanner strahlt einen Strahlfächer in einer Abtastebene 13 ab, um ein 2D-Profil 14 zu erfassen, und rotiert zusätzlich um eine Achse 15, um aus einer Vielzahl von 2D-Profilen 14 ein 3D-Abbild bzw. Reflexionsbild seiner Umgebung zu erzeugen. Ein Beispiel eines derartigen 3D-Laserscanners ist das Produkt mit der Markenbezeichnung VZ^{®} 400 der Firma RIEGL Laser Measurement Systems GmbH in Horn, Österreich.

Ein 3D-Laserscanner 10 kann einerseits so angesteuert werden, daß er genau einen Laserstrahl 12 in Richtung auf einen Satelliten sᵢ aussendet, um die Sichtlinie 4 wie erörtert abzutasten. Bevorzugt wird der 3D-Laserscanner 10 jedoch so betrieben, daß er zunächst in einem Zug ein 3D-Reflexionsbild seiner Umgebung erstellt, in welchem anschließend - z.B. in einem Post-Processing-Schritt - mit Hilfe an sich bekannter Verarbeitungsverfahren die Sichtlinie 4 zwischen Empfänger 2 und Satelliten sᵢ auf Reflexions- bzw. Hindernisfreiheit überprüft wird.

In einer vereinfachten Ausführungsform dieser Verfahrensvariante kann anstelle eines 3D-Laserscanners auch ein vom Fahrzeug 7 bewegter 2D-Laserscanner verwendet werden, welcher zwar nur ein 2D-Profil 14 in einer einzigen vorgegebenen Abtastebene 13 erzeugen kann, jedoch durch die Bewegung des Fahrzeugs 7 selbst die Umgebung abtastet und so zahlreiche 2D-Profile 14 erzeugt, aus denen sich wieder ein 3D-Reflexionsbild der Umgebung ergibt. Ein Beispiel eines derartigen 2D-Laserscanners ist das Produkt mit der Markenbezeichnung VQ^{®} 250 der Firma RIEGL Laser Measurement Systems GmbH in Horn, Österreich.

Zur korrekten Ausrichtung des Laserscanners 10 auf einen Satelliten sᵢ bzw. des von ihm erzeugten 3D-Reflexionsbildes auf das Referenzkoordinatensystem 3 aller Satelliten sᵢ ist die Anlage 11 mit einer Einrichtung 16 zur Messung der Orientierung des Laserscanners 10 gegenüber dem Referenzkoordinatensystem 3 ausgestattet. Die Einrichtung 16 kann im einfachsten Fall eine Wasserwaage mit Kompaß sein. Bevorzugt wird die Einrichtung 16 durch elektronische Gravitations- und Magnetfeldsensoren in Form einer sog. Trägheitsmeßeinheit (inertial measurement unit, IMU) gebildet, wie in der Technik bekannt.

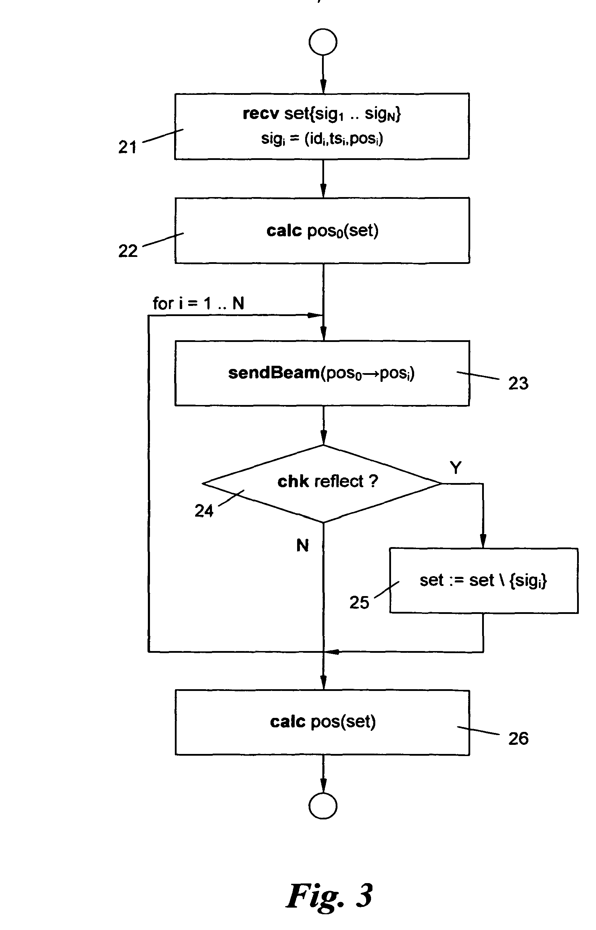

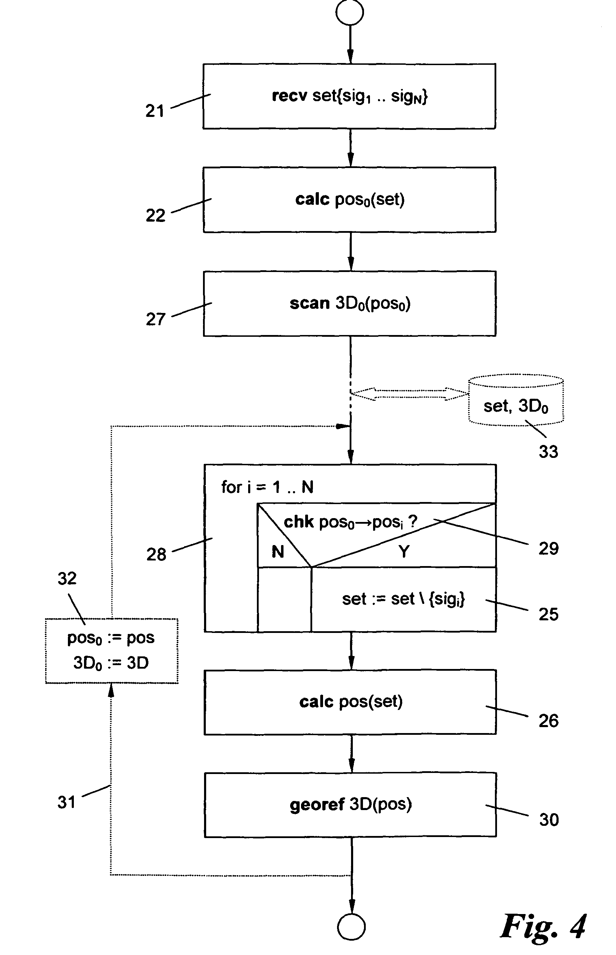

Die Verfahrensvarianten der Erfindung werden nun unter Bezugnahme auf die Fig. 3 und 4 im Detail erläutert. Fig. 3 zeigt die erstgenannte Ausführungsform des Verfahrens, welche ein gerichtetes Aussenden des Laserstrahls 12 zum Ort posᵢ eines Satelliten sᵢ verwendet.

In einem ersten Schritt 21 werden im Empfänger 2 die Sendesignale sigᵢ aller Satelliten sᵢ als Satz set{sig₁, sig₂, ...sig_{N}} (i = 1..N) empfangen. Anschließend wird in Schritt 22 aus dem Satz set eine vorläufige Position pos₀ des Empfängers 2 berechnet.

Die nachfolgenden Schritte 23 - 25 werden für alle N Sendesignale sigᵢ durchgeführt. In Schritt 23 wird ein Laserstrahl 12 von der vorläufigen Position pos₀ zur Position posᵢ, wie sie im Sendesignal sigᵢ eines Satelliten sᵢ angegeben ist, gesandt. In Schritt 24 wird festgestellt, ob der Laserstrahl 12 reflektiert wird (Y) oder nicht (N). Falls ja, wird in Schritt 25 das Sendesignal sigᵢ dieses Satelliten sᵢ aus dem Satz set ausgeschlossen.

Schließlich wird in Schritt 26 die Position pos des Empfängers 2 neubestimmt, und zwar durch Auswerten des in den Schritten 23 - 25 überarbeiteten Satzes set, welcher nun nur mehr jene Sendesignale sigᵢ enthält, in deren Richtung keine Reflexion festgestellt worden ist.

Falls gewünscht, könnte das Verfahren zur Verifizierung der Positionsbestimmung oder Verfeinerung iteriert werden, indem die so ermittelte Position pos nochmals als "vorläufige" Position pos₀ eingesetzt und die Schritte 23 - 26 neuerlich durchlaufen werden (in Fig. 3 nicht gezeigt).

Fig. 4 zeigt die zweitgenannte Verfahrensvariante, bei welcher nach den ersten beiden Schritten 21, 22 der Bestimmung der vorläufigen Position pos₀ zunächst ein ganzer Bereich der Umgebung des Empfängers 2 mit Hilfe des Laserscanners 10 gescannt wird, um ein 3D-Reflexionsbild 3D₀ des Umgebungsbereichs zu erzeugen, siehe Schritt 27.

In einer anschließenden Schleife 28 über alle Sendesignale sigᵢ (i = 1..N) wird für jede mögliche Sichtlinie pos₀→posᵢ im 3D-Reflexionsbild 3D₀ überprüft (Schritt 29), ob eine Hindernisreflexion vorliegt (Y) oder nicht (N). Bejahendenfalls wird jeweils das Sendesignal sigᵢ dieses Satellitens sᵢ aus dem Satz set ausgeschlossen, siehe Schritt 25.

In Schritt 26 wird nun wieder die Position pos des Empfängers 2 auf Grundlage des zuvor bereinigten Satzes set berechnet. Optional kann das 3D-Reflexionsbild 3D₀ auf die neue Position pos im Koordinatensystem 3 bezogen werden, um ein korrekt georeferenziertes 3D-Reflexionsbild 3D zu erzeugen (Schritt 30).

Falls gewünscht, können die Schritte 28, 26 und 30 in einer Schleife 31 iteriert werden, wobei jeweils die zuletzt bestimmte Position pos bzw. das zuletzt georeferenzierte 3D-Reflexionsbild 3D wieder als "vorläufige" Position pos₀ bzw. "vorläufiges" 3D-Reflexionsbild 3D₀ eingespeist werden, siehe Schritt 32.

Die Verfahrensvariante von Fig. 4 eignet sich besonders für eine örtliche und/oder zeitliche Abtrennung der rechenintensiven Auswerteschritten 25, 26, 28 - 32 von den vor Ort durchzuführenden Schritten 21, 22 und 27. Zu diesem Zweck können die Ausgangsrohdaten set und 3D₀ der Schritte 21, 22, 27 in einem Speicher 33 der Anlage 11 für die spätere und/oder räumlich entfernte Verarbeitung der Schritte 25, 26, 28 - 32 protokolliert bzw. zwischengespeichert werden.

Die Erfindung ist nicht auf die dargestellten Ausführungsbeispiele beschränkt, sondern umfaßt alle Varianten und Modifikationen, die in den Rahmen der angeschlossenen Ansprüche fallen.

## Patentansprüche

1. Verfahren zum Erzeugen eines auf eine Position (pos) eines Empfängers (2) in einem globalen Satellitennavigationssystem (1) georeferenzierten 3D-Reflexionsbildes (3D) eines Umgebungsbereiches des Empfängers (2) durch Laserscannen des Umgebungsbereiches und Auswerten von im Empfänger (2) empfangenen Sendesignalen (sigᵢ) mehrerer Satelliten (sᵢ), **gekennzeichnet durch** die Schritte
Bestimmen (21, 22) einer vorläufigen Position (pos₀) des Empfängers (2),
Aussenden (23, 27) eines Laserstrahls (12) von der vorläufigen Position (pos₀) in Richtung (4) zumindest eines empfangbaren Satelliten (sᵢ) und Feststellen (24, 27), ob der Laserstrahl (12) **durch** ein Hindernis reflektiert wird, und
Bestimmen (26) der Position (pos) des Empfängers (2) **durch** Auswerten der Sendesignale (sigᵢ) nur jener Satelliten (sᵢ), in deren Richtung (4) keine Reflexion festgestellt wird,
wobei das Aussenden des Laserstrahls und Feststellen einer Reflexion **durch** das genannte Laserscannen (27) des Umgebungsbereichs erfolgt, um ein auf die vorläufige Position (pos₀) bezogenes 3D-Reflexionsbild (3D₀) des Umgebungsbereichs zu erzeugen, in welchem anschließend festgestellt wird (29), ob die genannte Reflexion in Richtung (4) eines Satelliten (sᵢ) vorliegt, und
wobei das 3D-Reflexionsbild (3D₀) nach dem Bestimmen (26) der Position (pos) auf diese georeferenziert wird (3D, 30).

2. Verfahren nach Anspruch 1, **dadurch gekennzeichnet, daß** das Auswerten (29) des 3D-Reflexionsbildes (3D₀) und Bestimmen (26) der Position (pos) in einer von den vorherigen Schritten (21, 22, 27) abgesonderten Nachverarbeitung durchgeführt werden.

3. Verfahren nach Anspruch 1 oder 2, **dadurch gekennzeichnet, daß** das Aussenden (23, 27) des Laserstrahls (12) und Feststellen einer Reflexion mit Hilfe eines 3D-Laserscanners (10) erfolgt.

4. Verfahren nach einem der Ansprüche 1 bis 3, **dadurch gekennzeichnet, daß** die vorläufige Position (pos₀) in an sich bekannter Weise durch Auswerten der Sendesignale (sigᵢ) aller empfangbaren Satelliten (sᵢ) bestimmt wird.

5. Anlage zum Erzeugen eines auf eine Position in einem globalen Satellitennavigationssystem georeferenzierten 3D-Reflexionsbildes eines Umgebungsbereiches, mit einem Empfänger (2), der Sendesignale mehrerer Satelliten empfängt und auswertet, um daraus seine Position zu bestimmen, **dadurch gekennzeichnet,**
**daß** die Anlage (11) einen Laserscanner (10) aufweist und dazu ausgebildet ist, dessen Laserstrahl (12) in Richtung (4) zumindest eines Satelliten (sᵢ) auszurichten, um eine Strahlreflexion durch ein Hindernis in dieser Richtung festzustellen,
wobei der Empfänger (2) die Sendesignale (sigᵢ) nur jener Satelliten (sᵢ) auswertet, in deren Richtung (4) der Laserscanner (10) keine Strahlreflexion feststellt, und
wobei der Laserscanner (10) das genannte 3D-Reflexionsbild (3D₀) des Umgebungsbereichs erzeugt, um darin die genannte Strahlreflexion in Richtung (4) eines Satelliten (sᵢ) festzustellen, und dieses dann auf die so ermittelte Position (pos) georeferenziert.

6. Anlage nach Anspruch 5, **dadurch gekennzeichnet, daß** sie mit einem Speicher (33) versehen ist, welcher die vom Empfänger (2) empfangenen Sendesignale (sigᵢ) und die vom Laserscanner (10) erzeugten 3D-Reflexionsbilder (3D₀) für eine spätere gemeinsame Auswertung im Empfänger (2) protokolliert.

7. Anlage nach Anspruch 5 oder 6, **dadurch gekennzeichnet, daß** der Laserscanner (10) ein 3D-Laserscanner ist, welcher mit einer Einrichtung (16) zur Messung seiner Orientierung ausgestattet ist.

## Claims

1. Method for generating a 3D reflection image (3D) georeferenced to a position (pos) of a receiver (2) in a global satellite navigation system (1) of a surrounding area of the receiver (2) by laser scanning of the surrounding area and evaluation of transmission signals (sigᵢ) of multiple satellites (sᵢ) received in the receiver (2), **characterised by** the steps
determining (21, 22) a preliminary position (pos₀) of the receiver (2),
emitting (23, 27) a laser beam (12) from the preliminary position (pos₀) in the direction (4) of at least one receivable satellite (sᵢ) and determining (24, 27) whether the laser beam (12) is reflected by an obstruction, and
determining (26) the position (pos) of the receiver (2) by evaluating the transmission signals (sigᵢ) only of those satellites (sᵢ), in the direction (4) of which no reflection is determined,
wherein the emission of the laser beam and determination of a reflection is conducted by the said laser scanning (27) of the surrounding area in order to generate a 3D reflection image (3D₀) of the surrounding area in relation to the preliminary position (pos₀), in which it is subsequently determined (29) whether the said reflection lies in the direction (4) of a satellite (sᵢ), and
wherein the 3D reflection image (3D₀) is georeferenced (3D, 30) to the position (pos) after determination (26) thereof.

2. Method according to claim 1, **characterised in that** the evaluation (29) of the 3D reflection image (3D₀) and determination (26) of the position (pos) are conducted in a subsequent processing action separate from the previous steps (21, 22, 27).

3. Method according to claim 1 or 2, **characterised in that** the emission (23, 27) of the laser beam (12) and determination of a reflection are conducted by means of a 3D laser scanner (10).

4. Method according to one of claims 1 to 3, **characterised in that** the preliminary position (pos₀) is determined in a manner known per se by evaluating the transmission signals (sigᵢ) of all receivable satellites (sᵢ).

5. Assembly for generating a 3D reflection image of a surrounding area georeferenced to a position in a global satellite navigation system with a receiver (2), which receives and evaluates transmission signals of multiple satellites in order to determine its position therefrom, **characterised in that**
the assembly (11) has a laser scanner (10) and is configured to orient the laser beam (12) thereof in the direction (4) of at least one satellite (sᵢ) in order to determine a beam reflection by an obstruction in this direction,
wherein the receiver (2) evaluates the transmission signals (sigᵢ) only of those satellites (sᵢ) in the direction (4) of which the laser scanner (10) determines no beam reflection, and
wherein the laser scanner (10) generates the said 3D reflection image (3D₀) of the surrounding area in order to determine therein the said beam reflection in the direction (4) of a satellite (sᵢ), and then georeferences this to the position (pos) thus determined.

6. Assembly according to claim 5, **characterised in that** it is provided with a storage means (33), which records the transmission signals (sigᵢ) received by the receiver (2) and the 3D reflection images (3D₀) generated by the laser scanner (10) for a subsequent joint evaluation in the receiver (2).

7. Assembly according to claim 5 or 6, **characterised in that** the laser scanner (10) is a 3D laser scanner, which is equipped with a means (16) for measuring its orientation.

## Revendications

1. Procédé permettant de générer une image de réflexion 3D (3D), référencée géographiquement à une position (pos) d'un récepteur (2) dans un système mondial de navigation satellitaire (1), d'un domaine environnant du récepteur (2) par balayage avec un laser du domaine environnant et permettant d'évaluer des signaux d'émission (sigᵢ) reçus dans le récepteur (2) et émis par plusieurs satellites (sᵢ), **caractérisé par** les étapes consistant à :
déterminer (21, 22) une position provisoire (pos₀) du récepteur (2),
émettre (23, 27) un rayon laser (12) depuis la position provisoire (pos₀) en direction (4) d'au moins un satellite pouvant être reçu (sᵢ) et déterminer (24, 27) si le rayon laser (12) est réfléchi par un obstacle, et
déterminer (26) la position (pos) du récepteur (2) par le biais de l'évaluation des signaux d'émission (sigᵢ) des seuls satellites (sᵢ) dans la direction (4) desquels aucune réflexion n'a été déterminée,
l'émission du rayon laser et la détermination d'une réflexion ayant lieu par le biais dudit balayage par laser (27) du domaine environnant afin de générer une image de réflexion 3D (3D₀) du domaine environnant, liée à la position provisoire (pos₀) et dans laquelle il est déterminé ensuite (29) si ladite réflexion est présente en direction (4) d'un satellite (sᵢ), et
après la détermination (26) de la position (pos), l'image de réflexion 3D (3D₀) étant référencée géographiquement à cette position (3D, 3₀).

2. Procédé selon la revendication 1, **caractérisé en ce que** l'évaluation (29) de l'image de réflexion 3D (3D₀) et la détermination (26) de la position (pos) sont effectuées dans un traitement ultérieur séparé des étapes précédentes (21, 22, 27).

3. Procédé selon la revendication 1 ou 2, **caractérisé en ce que** l'émission (23, 27) du rayon laser (12) et la détermination d'une réflexion ont lieu à l'aide d'un dispositif de balayage à laser 3D (10).

4. Procédé selon l'une des revendications 1 à 3, **caractérisé en ce que** la position provisoire (pos₀) est déterminée d'une manière connue en soi en évaluant les signaux d'émission (sigᵢ) de tous les satellites (sᵢ) pouvant être reçus.

5. Installation pour générer une image de réflexion 3D d'un domaine environnant référencée géographiquement sur une position dans un système mondial de navigation satellitaire, comportant un récepteur (2) qui reçoit et évalue des signaux d'émission de plusieurs satellites afin de déterminer sa position à partir de ces signaux, **caractérisée en ce que**
l'installation (11) comprend un dispositif de balayage à laser (10) et est conçue pour diriger le rayon laser (12) dudit dispositif de balayage en direction (4) d'au moins un satellite (sᵢ) afin de déterminer une réflexion du rayon due à un obstacle dans cette direction,
le récepteur (2) évaluant les signaux d'émission (sigᵢ) des seuls satellites (si) dans la direction desquels (4) le dispositif de balayage à laser (10) ne détermine pas de réflexion de rayon, et
le dispositif de balayage à laser (10) générant ladite image de réflexion 3D (3D₀) du domaine environnant afin de déterminer dans celle-ci ladite réflexion de rayon en direction (4) d'un satellite (sᵢ) et, ensuite, il référencie géographiquement ladite image sur la position (pos) ainsi déterminée.

6. Installation selon la revendication 5, **caractérisée en ce qu'**elle est équipée d'une mémoire (33) qui enregistre un relevé sur les signaux d'émission (sigᵢ) reçus par le récepteur (2) et sur l'image de réflexion 3D (3D₀) générée par le dispositif de balayage à laser (10) en vue d'une évaluation ultérieure dans le récepteur (2).

7. Installation selon la revendication 5 ou 6, **caractérisée en ce que** le dispositif de balayage à laser (10) est un dispositif de balayage 3D qui est équipé d'un dispositif (16) pour mesurer son orientation.
